# FASCICULE DE BREVET EUROPEEN

(11) **EP 0 847 510 B1**
(45) Date de publication et mention de la délivrance du brevet: **16.04.2003**
(21) Numéro de dépôt: 96926995.0
(22) Date de dépôt: 29.08.1996
(51) Int. Cl.: F16K 15/14

(54) **VALVE**
VENTIL
VALVE

(30) Priorité: 01.09.1995 CH 249495
(43) Date de publication de la demande: 17.06.1998
(73) Titulaire: Climes Conseil, Claude Liardet, 1110 Morges (CH)
(72) Inventeur: LIARDET, Claude, CH-1170 Aubonne (CH)
(74) Mandataire: Ganguillet, Cyril
(86) Numéro de dépôt international: CH9600298
(87) Numéro de publication internationale: WO97009552

(56) Documents cités:
- EP-A- 0 094 813
- DE-A- 2 304 274
- GB-A- 2 064 072
- US-A- 3 113 586

## Description

La présente invention se rapporte à une valve utilisable dans des conduits d'écoulement de flux de différents types. Cette valve présente des propriétés améliorées en ce qui concerne notamment son ouverture, permettant un passage maximal des fluides.

Il existe un besoin pour des valves de ce genre dans différents domaines, notamment dans la construction d'appareils médicaux, par exemple d'appareils de soins des voies respiratoires. Dans ce domaine on connaît des appareils qui ont un rôle thérapeutique dont le but est d'améliorer les fonctions respiratoires des patients (par exemple EP-A-0565489) tout en dispensant un médicament sous forme de gouttelettes ou de particules dans le flux d'air aspiré. Ces appareils comportent divers conduits reliés à une embouchure et qui sont munis de valves pour permettre au patient d'effectuer une alternance d'aspiration et d'expiration.

Pour que ces appareils aient une bonne efficacité, les valves destinées au passage du médicament lors de l'inspiration doivent laisser, en position ouverte, une section maximale au passage du flux d'air et se fermer rapidement et complètement dès que l'effort d'aspiration cesse. En outre la disposition des organes de la valve doit être telle que l'écoulement ne soit pas perturbé, en particulier reste laminaire et évite le dépôt du médicament sur les parties de la valve ou les parois du conduit.

On s'est aperçu qu'aucune valve existante ne satisfaisait dans toute la mesure désirable à ces conditions. En particulier des essais ont mis en évidence avec les systèmes existants des pertes de substance active allant jusqu'à 80 %, ce qui dans le cas de médicaments coûteux ou difficiles à faire parvenir en quantité suffisante sur le tissu cible, représente une perte économique importante.

La valve selon la présente invention est une valve comportant une armature en forme de cadre rigide et un obturateur constitué d'un ensemble de palettes reliées au cadre par les articulations pivotant autour de ces dernières lors de l'ouverture sous l'effet de l'entraînement du fluide.

Des valves de ce type sont décrites dans le brevet américain no 4,351,358 et dans les publications des demandes de brevet français nos 2 410 198 et 2 584 162, ainsi que dans les brevets US-A-3,113,586 et DE-A-23 04 274.

L'obturateur décrit dans le premier de ces documents comporte une armature pyramidale ajourée, l'obturateur étant constitué par un ensemble de palettes planes triangulaires, montées sur des articulations à ressort, autour de l'armature. En position de fermeture, chaque palette s'appuie contre un des flans de la pyramide.

Les deux documents français décrivent des obturateurs formés de deux palettes en forme de segment de cône ou de segment de cylindre, chacun relié au siège par une articulation à ressort. En position de fermeture, chaque palette repose sur la surface annulaire plane du siège et se trouve en contact avec l'autre palette.

Les obturateurs décrits dans ces trois documents, de construction plutôt compliquée, sont munis de ressorts extérieurs. Ils sont destinés à des conduites pour le passage de fluides industriels, mais ne conviennent pas pour des appareils de soin des voies respiratoires.

Une valve destinée à un appareil respiratoire est décrite dans le brevet belge no 1005924. Elle comporte une pièce sortie par découpage d'une seule plaque de matériau élastomère, d'épaisseur constante sur toute sa surface. Cette valve ne constitue pas à proprement parler un clapet anti-retour mais fonctionne dans les deux sens, avec des pressions de résistance différentes. A cet effet, la plaque découpée forme un disque central relié par une languette de liaison à un anneau qui l'entoure, la zone centrale du disque étant divisée en plusieurs parties par des fentes radiales. La plaque est montée dans la paroi d'un tube et il est prévu un siège rigide qui sert d'appui à la périphérie du disque. Ainsi, dans un sens, la languette plie et le disque s'écarte du siège, comme un clapet normal, tandis que dans l'autre sens la périphérie du disque central est appuyée contre le siège et les parties séparées par les fentes radiales plient sous l'effet de l'écoulement, de sorte qu'un certain débit peut passer avec une résistance plus forte que dans l'autre sens. Une telle valve ne permet pas de satisfaire les conditions mentionnées ci-dessus.

Les brevets US-A-3,113,586 et DE-A-23 04 274, qui montre une valve selon le préambule de la revendication 1, concernent des valves cardiaques qui ne répondent pas non plus aux conditions requises ci-dessus. En particulier, ces valves ne remplissent pas la condition de section maximale au passage d'un flux d'air.

Le but de la présente invention est donc de réaliser une valve qui pallie les inconvénients mentionnés ci-dessus tout en étant économique à la réalisation et pratique à l'emploi.

Dans ce but l'invention propose une valve comportant une armature en forme de cadre rigide et un obturateur constitué d'un ensemble de palettes à surfaces sensiblement planes, reliées au cadre par des articulations et pivotant autour de ces dernières lors de l'ouverture sous l'effet de l'entraînement du fluide, le cadre et les palettes formant une pièce d'un seul tenant dans laquelle les palettes sont des éléments en soi rigides et les articulations des zones élastiquement déformables, flexibles autour d'un axe rectiligne situé entre la palette et une portion correspondante du cadre, caractérisée en ce que les palettes sont conformées de manière à assurer la retenue dû fluide dans la position de fermeture par l'appui exclusif de leurs tranches les unes contre les autres.

Selon un premier mode d'exécution de la valve, le cadre et l'ensemble des palettes sont formés d'une même pièce en un matériau homogène, les articulations étant des zones minces de la dite pièce situées chacune entre une palette et une portion correspondante du cadre, le dit matériau homogène pouvant être une matière plastique cristalline, notamment le polyacétal.

Selon une seconde forme d'exécution de la valve, les articulations sont réalisées à l'aide d'un matériau élastique, en particulier un élastomère.

Le cadre peut être de forme circulaire ou de forme polygonale, ou de toute autre forme adéquate, et être réalisé d'une pièce avec un segment de conduit guidant le flux ou incorporé à un segment de conduit guidant le flux.

Les palettes peuvent notamment être de forme triangulaire ou les bords des palettes incurvés de manière à donner une impulsion hélicoïdale au flux en position ouverte.

Le segment de conduit peut présenter des logements destinés à recevoir les palettes en positions ouvertes.

La valve selon la présente invention peut avantageusement être utilisé dans des appareils médicaux, notamment des appareils respiratoires.

On va décrire ci-après, à titre d'exemples, diverses formes de réalisation possibles de l'objet de l'invention, et différentes variantes, en se référant au dessin annexé, dont
la figure 1 est une vue en coupe, selon la ligne I-I de la figure 2 montrant une première forme d'exécution de la valve,
la figure 2 est une vue en plan de cette première forme d'exécution,
les figures 3 et 4 sont des agrandissements respectivement des détails A et B de la figure 1,
les figures 5 et 6 sont des vues, respectivement en coupe par un plan diamétral et en plan, d'une deuxième forme d'exécution de la valve,
les figures 7 et 8 sont'des vues en coupe par un plan diamétral de deux autres formes d'exécution de la valve,
les figures 9 et 10 sont des vues en perspective schématiques montrant les valves des figures 1 à 8 en position respectivement ouverte et fermée,
les figures 11 à 13 sont des vues en plan schématiques montrant différentes variantes relatives au nombre et à la forme des palettes, pour un conduit de forme circulaire,
les figures 14 à 17 sont des vues en coupe schématiques montrant des formes d'exécution dans lesquelles la valve proprement dite est solidaire d'un segment de conduit, et
les figures 18 à 22 sont des vues en plan schématiques montrant différentes variantes relatives à la section du conduit.

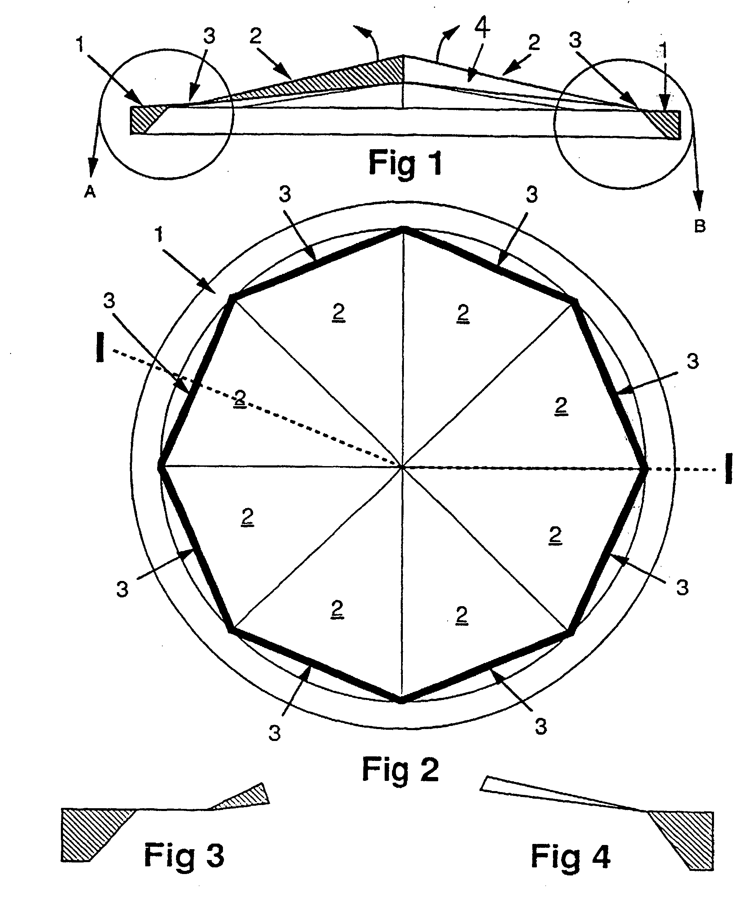

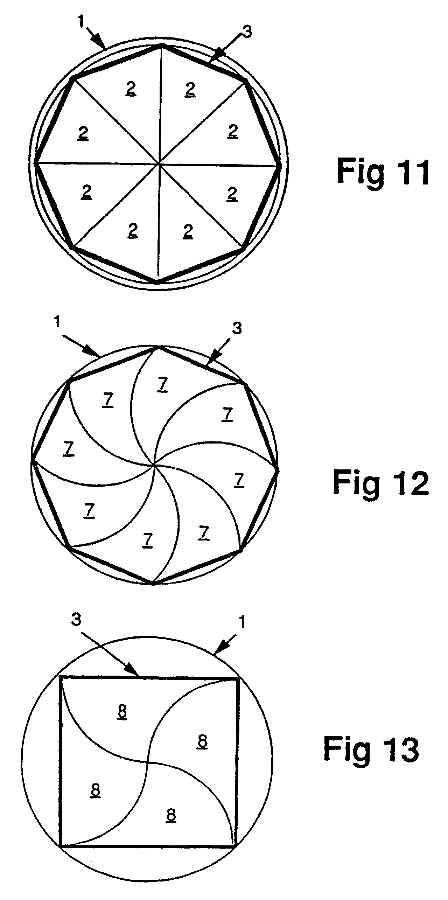

De manière générale, les valves selon les exemples des figures 1 à 22, à l'exception du mode d'exécution représenté à la figure 8, sont conçues de telle sorte que tous leurs éléments fonctionnels sont réalisés dans la même pièce, certaines parties étant élastiquement flexibles, alors que les autres sont rigides. On choisira pour cela des matériaux qui se prêtent à ces emplois, comme par exemple le polyacétal qui peut être très flexible dans ses parties minces et résistant dans ses parties épaisses. Ce matériau peut être mis en forme par injection. En général tout matériau plastique dur, non cassant, ayant des caractéristiques rappelant celles de l'acier peut convenir. Certains plastiques cristallins sont connus comme présentant de telles propriétés. D'ailleurs l'acier lui-même pourrait convenir dans certains cas, bien que l'usinage soit alors long et coûteux par rapport à une injection de plastique. Il est également possible d'obtenir les caractéristiques décrites ci-dessus en combinant plusieurs matériaux, en particulier comme illustré à la figure 8 qui présente des palettes réalisées en un matériau dur, la zone de flexion étant réalisée en un matériau élastique, comme par exemple un élastomère.

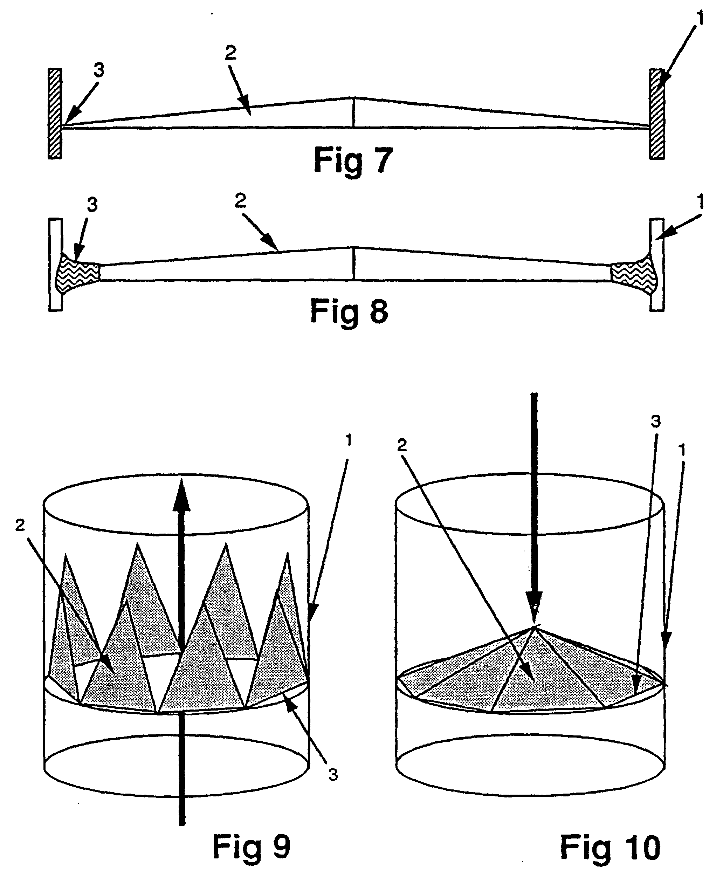

Les figures 1 à 7 montrent comment l'exécution en une pièce peut être réalisée. La valve comporte une partie fixe et rigide en forme de cadre circulaire 1 de profil trapézoïdal à partir de laquelle s'étendent des parties de forme triangulaire 2 jouant le rôle de palettes mobiles. Les palettes 2 sont rigides et se relient au cadre 1 par des zones minces 3 qui assurent l'articulation des palettes autour d'un axe coïncidant avec la base du triangle. En position fermée les palettes sont appuyées les unes contre les autres par leurs surfaces de tranche 4, et peuvent former une pyramide de faible hauteur (cas des palettes des valves des figures 1 à 6) ou être positionnées horizontalement (cas des palettes des valves des figures 7 et 8).

Dans la forme d'exécution selon les figures 1 et 2, le profil en long des palettes 2 est triangulaire. Leur épaisseur est maximale au point de rencontre central où toutes les palettes sont en contact, et décroît progressivement vers la périphérie de la valve. Les zones flexibles 3 ont en plan la forme de segments de disques.

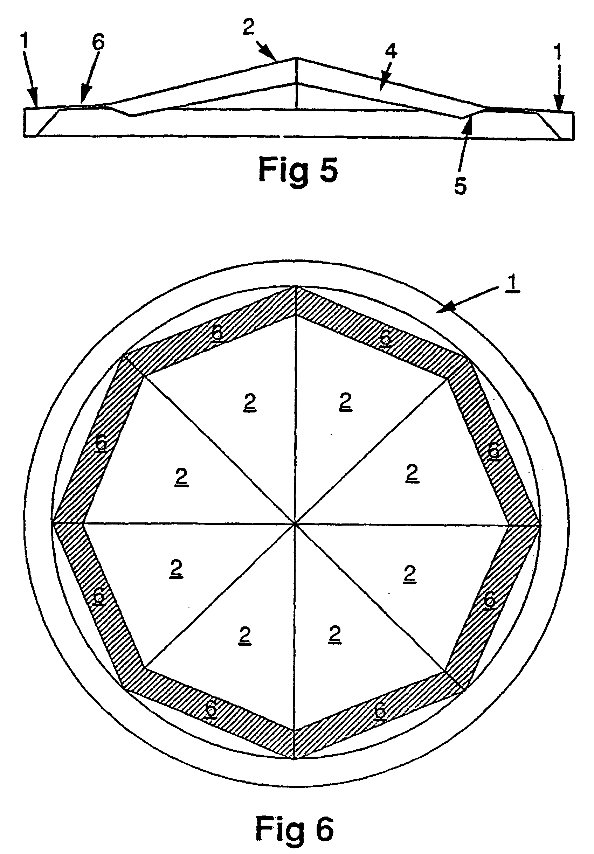

Dans la forme d'exécution selon les figures 5 et 6, l'épaisseur des palettes est constante depuis le point d'appui commun central jusqu'à la limite de la zone rigide. Des portions de surfaces en segments coniques 5 limitent les zones flexibles 6 qui sont elles-mêmes disposées selon des portions de cônes. Ces portions minces pourraient aussi se raccorder aux palettes au niveau de leur surface inférieure.

Les valves des deux premières formes d'exécution sont destinées à être montées dans un conduit de profil circulaire par tout moyen convenable : engagement à force du cercle 1 dans la face interne du conduit, serrage contre un épaulement au moyen d'un anneau à ressort, ou fixation au moyen d'un écrou, etc. Sous l'effet de la pression exercée par la masse d'air entraînée, les palettes pivotent autour de leur base et se placent dans la ligne du conduit, en offrant ainsi le minimum de résistance au passage de l'air. La figure 9 montre schématiquement ces positions tandis que les flèches de la figure 1 indiquent la direction du mouvement de pivotement. La valve se referme (figure 10) sous l'effet de l'élasticité des zones 3 dès que l'effet d'entraînement cesse.

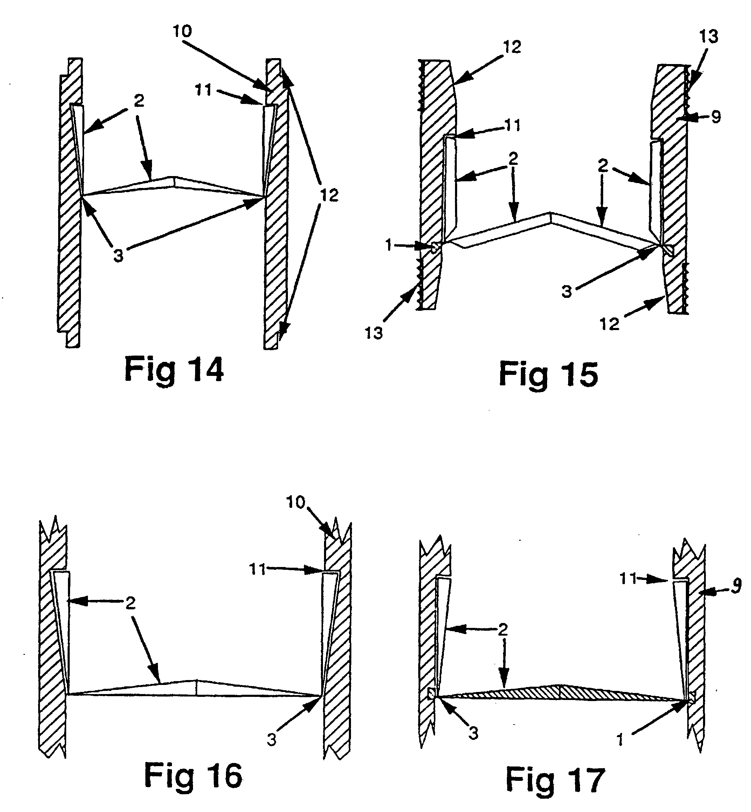

Au lieu que les palettes 2 soient de forme triangulaire en plan, elles peuvent aussi comme le montrent les figures 12 et 13 avoir la forme de triangles curvilignes 7 et 8, la figure 12 représentant une exécution à huit palettes comme les figures 1 à 4, et la figure 13 représentant une exécution à quatre palettes. La forme de triangle curviligne des figures 12 et 13 présente l'avantage de communiquer au flux d'air un mouvement de rotation en hélice autour de l'axe du conduit. Dans ce cas, les dégagements 11 dans l'épaisseur du conduit destinés au logement des palettes en position ouverte (décrits ci-après en regard des figures 14 à 17) ne seront de préférence pas prévus. Il est en effet dans ce cas nécessaire, qu'en position d'ouverture, les extrémités des palettes fassent saillie à l'intérieur du conduit pour communiquer le mouvement de rotation au flux.

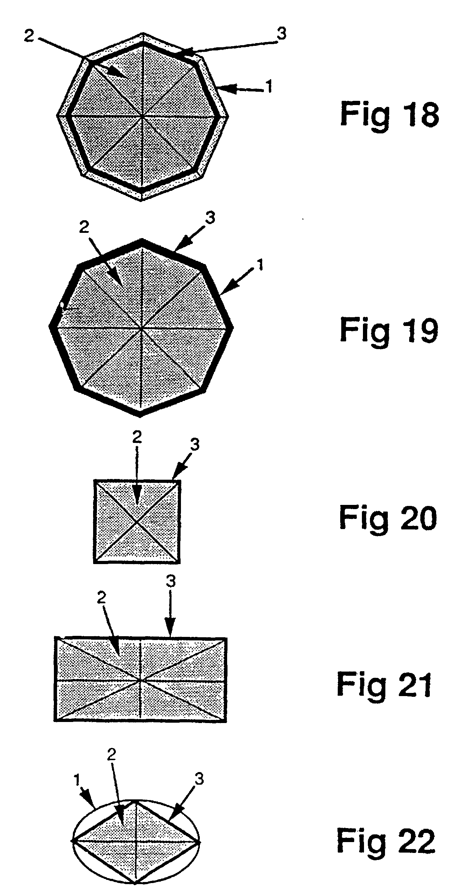

Dans le cas où le cadre rigide de la valve n'est pas de forme circulaire, mais de forme polygonale, comme par exemple octogonale (figures 18 et 19), carrée (figure 20) ou rectangulaire (figure 21), les zones minces 3 sont des bandes rectilignes étroites et non plus des segments de disques. Des valves avec de tels cadre polygonaux, destinées plus particulièrement à des conduits de formes polygonales correspondantes, permettent un meilleur passage du flux que dans le cas des conduits de profil circulaire. On peut bien entendu réaliser des valves avec des cadres présentant d'autres formes extérieures, comme par exemple elliptique (figure 22). Toutefois, dans ce dernier cas, le passage du flux est moins bon.

Enfin les figures 14 à 17 montrent des réalisations dans lesquelles un segment de conduit 9 ou 10 est associé à la valve proprement dite. Selon les figures 15 et 17 le cadre rigide de la valve est noyé dans l'épaisseur de ce segment de conduit 9, qui peut être d'un autre matériau que la valve, alors que selon les figures 14 et 16 c'est le segment de conduit 10 lui-même qui forme le cadre rigide de la valve. Dans les quatre exemples des dégagements 11 sont prévus dans l'épaisseur du conduit pour loger les palettes en position ouverte, afin qu'elles offrent le minimum de résistance au passage de l'air et évitent de retenir les gouttelettes de médicament incorporées au flux. On peut remarquer d'autre part que les valves des figures 14 et 16 offrent, lorsque les palettes sont en position d'ouverture, un meilleur passage au flux que les valves des figures 15 et 17. Les segments de conduit 9, 10 peuvent être pourvus d'organes de raccordement 12, 13 à leurs extrémités.

Les valves décrites ci-dessus présentent de nombreux avantages. En premier lieu elles sont d'une fabrication simple et rapide puisque la valve dans son ensemble peut être réalisée d'une seule pièce au cours d'une opération d'injection. Les moules peuvent être préparés avec toute la précision requise afin que les surfaces actives des différents éléments jouent leur rôle. En particulier les tranches des palettes peuvent être réalisées sous formes de surfaces complémentaires qui en position naturelle fermée s'appuient les unes contre les autres, de telle manière que l'ouverture se produise spontanément aussitôt que le flux d'air est dirigé dans le sens voulu (flèche de la figure 9).

La construction n'implique aucune partie faisant obstacle au passage de l'air en position ouverte ou modifiant sa direction ou ses caractéristiques.

Ainsi on se rend compte que la symétrie des formes et des caractéristiques aérodynamiques assure l'ouverture complète des palettes et leur fermeture instantanée seulement en fonction de la direction de déplacement du flux.

Le risque de dépôt de particules sur des parties produisant des chocs est évité et comme les couches limites ne sont pas perturbées, le flux reste laminaire ce qui est particulièrement important lors de l'emploi dans des appareils d'aide à la respiration à l'usage de patients souffrant d'asthme par exemple.

Toutefois les mêmes avantages peuvent aussi justifier l'emploi des valves décrites dans d'autres domaines médicaux, comme la cardiologie, ou de manière générale dans la conduction et la distribution de gaz, liquides, poudres, grains, etc. sur le plan industriel.

## Revendications

1. Valve comportant une armature en forme de cadre rigide (1) et un obturateur constitué d'un ensemble de palettes (2) à surfaces sensiblement planes, reliées au cadre par des articulations (3) et pivotant autour de ces dernières lors de l'ouverture sous l'effet de l'entraînement du fluide, le cadre et les palettes formant une pièce d'un seul tenant dans laquelle les palettes sont des éléments en soi rigides et les articulations des zones élastiquement déformables, flexibles autour d'un axe rectiligne situé entre la palette et une portion correspondante du cadre, **caractérisée en ce que** les palettes sont conformées de manière à assurer la retenue du fluide dans la position de fermeture par l'appui exclusif de leurs tranches les unes contre les autres.

2. Valve selon la revendication 1, **caractérisée en ce que** le cadre et l'ensemble des palettes sont formés d'une même pièce en un matériau homogène, les articulations étant des zones minces de la dite pièce situées chacune entre une palette et une portion correspondante du cadre.

3. Valve selon la revendication 2, **caractérisée en ce que** le dit matériau homogène est une matière plastique cristalline, notamment le polyacétal.

4. Valve selon la revendication 1, **caractérisée en ce que** les articulations sont réalisées à l'aide d'un matériau élastique, en particulier un élastomère.

5. Valve selon l'une quelconque des revendications 1 à 4, **caractérisée en ce que** le cadre est de forme circulaire.

6. Valve selon l'une quelconque des revendications 1 à 4, **caractérisée en ce que** le cadre est de forme polygonale.

7. Valve selon l'une quelconque des revendications précédentes, **caractérisée en ce que** les palettes sont de forme triangulaire.

8. Valve selon l'une quelconque des revendications 1 à 6, **caractérisée en ce que** les bords des palettes sont incurvés de manière à donner une impulsion hélicoïdale au flux en position ouverte.

9. Valve selon l'une quelconque des revendications 1 à 8, **caractérisée en ce que** le cadre est d'une pièce avec un segment de conduit guidant le flux.

10. Valve selon l'une quelconque des revendications 1 à 8, **caractérisée en ce que** le cadre est incorporé à un segment de conduit guidant le flux.

11. Valve selon l'une des revendications 9 ou 10, **caractérisée en ce que** le segment de conduit présente des logements destinés à recevoir les palettes en positions ouvertes.

12. Utilisation d'une valve selon l'une quelconque des revendications 1 à 11 dans des appareils médicaux, notamment des appareils respiratoires.

## Claims

1. A valve comprising a fitting in the form of a rigid frame (1) and a shutoff device consisting of an assembly of vanes (2), with surfaces which are approximately flat, connected to the frame by articulations (3) and pivoting about these articulations during the opening process under the effect of the introduction of the fluid, the frame and the vanes forming one single piece, in which the vanes are elements which are inherently rigid and the articulations are areas which are elastically deformable, being flexible about a rectilinear axis located between the vane and a corresponding portion of the frame, **characterised in that** the vanes are shaped in such a way as to ensure the retention of the fluid in the closed position by the exclusive support of their border edges against one another.

2. A valve according to claim 1, **characterised in that** the frame and the assembly of vanes are formed of the same piece in a homogenous material, the articulations being thin areas of the said piece, each located between a vane and a corresponding portion of the frame.

3. A valve according to claim 2, **characterised in that** the said homogenous material is a crystalline plastic material, polyacetal in particular.

4. A valve according to claim 1, **characterised in that** the articulations are created with the aid of an elastic material, and an elastomer in particular.

5. A valve according to any of claims 1 to 4, **characterised in that** the frame is circular in shape.

6. A valve according to any of claims 1 to 4, **characterised in that** the frame is polygonal in shape.

7. A valve according to any of the preceding claims, **characterised in that** the vanes are triangular in shape.

8. A valve according to any of claims 1 to 6, **characterised in that** the edges of the vanes are curved inwards in such a way as to provide a helicoidal thrust to the flow when in the open position.

9. A valve according to any of claims 1 to 8, **characterised in that** the frame forms one piece with a segment of the tube guiding the flow.

10. A valve according to any of claims 1 to 8, **characterised in that** the frame is incorporated with a segment of the tube guiding the flow.

11. A valve according to one of claims 9 or 10, **characterised in that** the segment of tube features recesses intended to accommodate the vanes in the open position.

12. The use of a valve according to any of claim 1 to 11 in medical equipment, and particular in respiratory equipment.

## Patentansprüche

1. Ventil mit einer Armatur in Form eines steifen Rahmens (1) und mit einem Verschlussteil, das aus einer Anordnung aus Plättchen (2) mit im wesentlichen ebenen Flächen besteht, die mit dem Rahmen über Gelenke (3) verbunden sind und die um diese während des Öffnens verschwenkbar sind auf Grund der Strömungswirkung eines Fluids, wobei der Rahmen und die Plättchen einstückig ausgebildet sind, bei dem die Plättchen in sich steife Bauteile sind und die Gelenke elastisch verformbare Bereiche sind, die um eine gerade Achse flexibel sind, welche sich zwischen einem Plättchen und einem zugehörigen Abschnitt des Rahmens erstreckt, **dadurch gekennzeichnet, dass** die Plättchen derart ausgestaltet sind, dass sie ein Zurückhalten des Fluids in der Verschlussstellung gewährleisten ausschließlich durch Abstützung ihrer Ränder aufeinander.

2. Ventil nach Anspruch 1, **dadurch gekennzeichnet, dass** der Rahmen und die Anordnung aus Plättchen aus einem einzigen Teil eines homogenen Materials bestehen, wobei die Gelenke aus dünnen Bereichen dieses Teils bestehen, deren jedes zwischen einem Plättchen und dem zugehörigen Abschnitt des Rahmens angeordnet ist.

3. Ventil nach Anspruch 2, **dadurch gekennzeichnet, dass** das homogene Material ein kristalliner Kunststoff ist, insbesondere Polyacetal.

4. Ventil nach Anspruch 1, **dadurch gekennzeichnet, dass** das homogene Material ein elastisches Material ist, insbesondere ein Elastomer.

5. Ventil nach einem der Ansprüche 1 bis 4, **dadurch gekennzeichnet, dass** der Rahmen eine kreisförmige Gestalt aufweist.

6. Ventil nach einem der Ansprüche 1 bis 4, **dadurch gekennzeichnet, dass** der Rahmen eine polygonale Gestalt aufweist.

7. Ventil nach einem der vorhergehenden Ansprüche, **dadurch gekennzeichnet, dass** die Plättchen eine dreieckige Gestalt aufweisen.

8. Ventil nach einem der Ansprüche 1 bis 6, **dadurch gekennzeichnet, dass** die Ränder der Plättchen derart gekrümmt sind, dass sie der Strömung in der Offenstellung einen schraubenförmigen Impuls erteilen.

9. Ventil nach einem der Ansprüche 1 bis 8, **dadurch gekennzeichnet, dass** der Rahmen einstückig mit einem die Strömung fördernden Leitungssegment ausgebildet ist.

10. Ventil nach einem der Ansprüche 1 bis 8, **dadurch gekennzeichnet, dass** der Rahmen in ein die Strömung förderndes Leitungssegment eingesetzt ist.

11. Ventil nach einem der Ansprüche 9 oder 10, **dadurch gekennzeichnet, dass** das leitungssegment Aussparungen aufweist zur Aufnahme der Plättchen in den Offenstellungen.

12. Verwendung eines Ventils nach einem der Ansprüche 1 bis 11 in medizinischen Geräten, insbesondere in Geräten zur Behandlung der Atemwege
